# EUROPEAN PATENT SPECIFICATION

(11) **EP 2 044 881 B1**
(45) Date of publication and mention of the grant of the patent: **20.08.2014**
(21) Application number: 08165624.1
(22) Date of filing: 01.10.2008
(51) Int. Cl.: A61B 5/022

(54) **Sphygmomanometer**
Sphygmomanometer
Sphygmomanomètre

(30) Priority: 03.10.2007 JP 2007260423
(43) Date of publication of application: 08.04.2009
(73) Proprietor: Japan Precision Instruments Inc., Shibukawa-shi Gumma 377-0293 (JP)
(72) Inventor: Kato, Junichi, Shibukawa-shi Gunma 377-0293 (JP)
(74) Representative: Betten & Resch

(56) References cited:
- JP-A- 2001 112 723
- JP-A- 2006 158 543
- JP-U- 62 130 606

## Description

### BACKGROUND OF THE INVENTION

### 1. Field of the invention

The present invention relates to a sphygmomanometer such as a wrist sphygmomanometer in which a body unit (sphygmomanometer body) and a cuff unit (cuff band) are integrally formed.

### 2. Description of the Related Art

As a sphygmomanometer in which a body unit and a cuff unit are integrally formed, a wrist sphygmomanometer mainly used by being wound on an arm is known.

The wrist sphygmomanometer is composed of the body unit and the cuff unit, with the body unit having an air pump, an exhaust valve, and a pressure sensor, as blood pressure measuring function parts, inside of a body case having a displayer for displaying a blood pressure , etc, and switches such as a power switch, etc. An entire body of the cuff unit is covered with an outer bag such as a cloth, etc, and a bladder (air bag) is arranged inside of a cuff spring made of a hard material.

In this case, as the sphygmomanometer, there is a type connecting the body unit and the cuff unit completely by a screw, etc, so as not to be detached from each other, and also there is a type connecting the body unit and the cuff unit so as to be detached from each other, for mainly maintenance and replacement of the cuff unit.

The wrist sphygmomanometer described in patent document 1 discloses the wrist sphygmomanometer of the type connecting the body unit and the cuff unit so as to be detached from each other as described above.

According to the sphygmomanometer described in the patent document 1, plural sets of engagement hooks and engagement holes are formed on a connecting surface of the body unit and the cuff unit, so as to be detachably engaged with each other, and by mutually engaging these engagement hooks and engagement holes by using elasticity of the engagement hooks, the body unit and the cuff unit are connected to each other, and also an air passage connection hole on the body unit side and an air passage connection hole on the cuff unit side are connected to each other.

(Patent document 1) Japanese Patent Laid Open Publication NO.08-191804
JP 2001112723 A discloses an integrated sphygmomanometer with a structure being easy to fit over various shapes of a part to be inspected, allowing a sphygmomanometer body to be easily attached to and removed from a blood stopping cuff without the possibility of inadvertent disconnection, and being easy to be miniaturized. The integrated sphygmomanometer provides a connecting hook disposed on a blood stopping cuff for connection to a connecting hole disposed in the lower surface of the sphygmomanometer body is fixed to a locking member from the back of the hook.
As further prior art reference is made to JP 62130606 U.

### SUMMARY OF THE INVENTION

Incidentally, patent document 1 only discloses the wrist-wound sphygmomanometer in which by pressing the body unit and the cuff unit against each other in a direction orthogonal to the connecting surface of them, the engagement hooks are engaged with the engagement holes by utilizing bending property of each engagement hook. Therefore, when a force strong in a separating direction is added, there is a possibility that the engagement hook is bent to the opposite side, and each engagement hook and each engagement hole are disengaged from each other, resulting in unexpected detachment of the cuff unit from the body unit.

In addition, it is necessary to connect the air passage connection hole on the body unit side and the air passage connection hole on the cuff unit side, simultaneously with an operation of inserting the engagement hook into the engagement hole. Therefore, positioning of the air passage connection hole must be performed, simultaneously with the positioning of the engagement hook and the engagement hole. Accordingly, there is a possibility that the body unit and the cuff unit are hardly connected to each other.

In consideration of the above-described circumstances, the present invention is provided, and an object of the present invention is to provide the sphygmomanometer capable of making it difficult to detach the cuff unit from the body unit when connected to each other once, capable of easily connecting the air passages of them, and capable of easily performing the maintenance and replacement of the cuff unit.

The invention provides a sphygmomanometer of claim 1. It is composed of a body unit and a cuff unit connected to a bottom face of this body unit, in which plural sets of engagement hooks and engagement holes engaged with one another detachably are formed on one and the other connecting surface of the body unit and the cuff unit, and by engagement of the engagement hooks and the engagement holes with one another, the body unit and the cuff unit are connected to each other, and simultaneously an air passage connection hole on the body unit side and an air passage connection hole on the cuff unit side are connected to each other,
each engagement hole having through hole parts through which the engagement hooks can be inserted and extracted into/from the engagement holes by relatively moving the body unit and the cuff unit in a direction orthogonal to the connecting surface; and
a slipoff preventive hole with locking wall for preventing coming off of the engagement hooks, by relatively sliding the body unit and the cuff unit in a first direction parallel to the connecting surface, with the engagement hooks inserted into the through holes,
the body unit and the cuff unit having a lock mechanism of inhibiting the movement of the body unit and the cuff unit in a second direction opposite to the first direction, with the engagement hooks moved to the slipoff preventive hole parts of the engagement holes, and
an air passage connection hole on the body unit side and an air passage connection hole on the cuff unit side being provided, with a connecting direction determined so that the body unit and the cuff unit are engaged with each other by relatively sliding them in the first direction, and they are disengaged by relatively sliding them in the second direction.

The lock mechanism is composed of a set of a lock claw and a lock hole provided at one side and the other side of the connecting surface of the body unit and the cuff unit, with the lock hole having:
an insertion hole part with locking wall for receiving the lock claw by utilizing its bending property, when the body unit and the cuff unit are relatively moved in a direction orthogonal to the connecting surface; and
a locking hole part for inhibiting the movement of the body unit and the cuff unit in a second direction by abutment of the body unit and the cuff unit on the locking wall of the insertion hole part with locking wall, when the body unit and the cuff unit are relatively slid in the first direction parallel to the connecting surface and inserting the engagement hooks into the engagement holes with locking wall to receive the lock claw and in this state when the body unit and the cuff unit are relatively moved in the second direction.

Advantageously,the first direction and the second direction are set in an axial direction of the cuff unit.

Advantageously, there is provided an air connector having a connection hole, being an air passage connection hole on the body unit side formed at one end side of the connecting surface of the body unit, and the connection hole connected to an air pump, an exhaust valve, and a pressure sensor equipped in the body unit formed at the other end side.

Advantageously the air connector is constituted of a tubular component having a first connection hole connected to the air pump and the exhaust valve equipped in the body unit and a second connection hole connected to the pressure sensor equipped in the body unit in an outer periphery, with longitudinal one end having the connection hole as the air passage connection hole, and the other end closed, and the air connector with this structure is disposed approximately parallel to an axial direction of the cuff unit.

Advantageously a protruding part that protrudes toward a curved inner face is provided in approximately center position in the peripheral direction of a curve-shaped cuff spring constituting the cuff unit, and by providing this protruding part, the air connector is accommodated in a recess portion formed on an outer surface side of the cuff spring.

### Advantage of the Invention

According to the first invention, by performing positioning of the engagement hooks to the through hole parts of the engagement holes, while the connecting surface of the body unit and the connecting surface of the cuff unit are approached to each other and pressed against each other (this is called "first action"), the engagement hooks can be inserted into the engagement holes through the through holes parts. In addition, by making the body unit and the cuff unit relatively slide in the first direction, with the engagement hooks inserted into the through hole parts (this is called "second action"), the engagement hooks can be moved to the slipoff preventive hole part with locking wall. Therefore, coming-off of each engagement hook can be prevented by the engagement of the engagement hook and the engaging wall.

By operation of the lock mechanism in this stage, the engagement hook can not return to the through hole part, thereby surely maintaining a coming-off prevention state. In addition, by operation of making the body unit and the cuff unit relatively slide in the first direction, the air passage connection hole on the body unit side and the air passage connection hole on the cuff unit side can be connected to each other, thus completely connecting the body unit and the cuff unit.

In this case, by performing the second operation after performing the first operation, the coming-off of the engagement hook can be surely prevented. Therefore, as long as the lock mechanism is not released, the connection of the body unit and the cuff unit is not released, even if trying to separate them with a strong force. Namely, even if a release preventing capability in the direction orthogonal to the connecting surface is added between the body unit and the cuff unit, the body unit and the cuff unit are not separated from each other unless the engagement hook moves to the through hole part, because the engagement hook is inserted up to the coming-off prevention part. Accordingly, in order to extract the engagement hook from the engagement hole, two actions in different directions (the action reverse to the second action and the action reverse to the first action) must be performed sequentially, and if the body unit and the cuff unit are connected to each other once, they are hardly detached from each other.

In addition, when the second action of making the body unit and the cuff unit relatively slide in a direction parallel to the connecting surface is performed after the first action of inserting the engagement hook into the engagement hole, the air passage connection hole on the body unit side is connected to the air passage connection hole on the cuff unit side for the first time. Therefore, the air passage connection holes are connected to each other, while guiding sliding operation of the engagement hook. This makes it possible to easily connect air circuits. Namely, positioning of the body unit and the cuff unit is necessary only when the positioning of the engagement hook and the engagement hole is performed, and when the air passage connection holes are connected to each other, the connection can be completed only by performing the operation of automatic guide. Accordingly, connecting work of the body unit and the cuff unit can be facilitated.

In addition, when the connection between the body unit and the cuff unit is released, first, the lock of the lock mechanism is released. Then, the body unit and the cuff unit are made to slide in the second direction. Then, the engagement hook moves to the through hole part form the slipoff preventive hole part with locking wall. Moreover, simultaneously with this movement of the engagement hook, the air passage connection hole on the body unit side is detached from the air passage connection hole on the cuff unit side. Accordingly, in this state, when the body unit and the cuff unit are relatively moved in a releasing direction orthogonal to the connecting surface, the engagement hook can be extracted from the engagement hole, and the connection between the body unit and the cuff unit can be released. Therefore, for example when the maintenance of the cuff unit is performed or the replacement thereof is performed, by separating the cuff unit from the body unit and the maintenance and replacement work can be easily performed.

According to the second invention, only by forming the combination of the lock claw and the lock hole, in the same way as the combination of the engagement hook and the engagement hole, the lock mechanism can be constituted. Therefore, designing/processing can be facilitated. Also, insertion of the lock claw into the lock hole can be performed, simultaneously with inserting o0peration of the engagement hook. Therefore, there is no trouble in operation generated. Also, by utilizing the bending property of the lock claw itself, the lock claw is inserted into an insertion hole part with locking wall of the lock hole. Therefore, temporary locking action of the locking wall and the lock claw can be expected. This is useful for smoothly performing transition form the first action to the second action.

According to the third invention, the direction of a sliding action of the body unit and the cuff unit for engagement/disengagement between the engagement hook and the engagement hole is set in the axial direction of the cuff unit. Therefore, a connection structure tolerant to a lateral motion orthogonal to the axial direction (vertical direction) can be realized. This is because an idle dimension of the engagement hole (particularly the slipoff preventive hole with locking wall) with respect to the engagement hook can be made small as much as possible, in the lateral direction orthogonal to the direction of the sliding motion.

Therefore, for example, when the sphygmomanometer is used, with the cuff unit actually fitted to a wrist, positional adjustment is performed in many cases while rotating the cuff unit around the wrist. At that time, a force is added laterally to the body unit in many cases, but in such an adjustment operation also, the connection state between the body unit and the cuff unit can be maintained without rattling.

In addition, when the body unit and the cuff unit are slid in the vertical direction (axial direction), a length of the connecting surface of the body unit and the cuff unit can be set longer in the vertical direction than in the lateral direction. Therefore, longer sliding strokes can be obtained. This is because the cuff unit has a curvature in the direction (lateral direction) orthogonal to the axial direction so as to be wound around the wrist, and therefore the length of the connecting surface can be hardly set to be larger in the lateral direction. Accordingly, since the longer sliding strokes can be obtained, an engaging width (engaging margin) of the engagement hook and the engaging wall can be set larger, and this contributes to increasing an engaging strength. In addition, an engaging length between the air passage connection hole on the body unit side and the air passage connection hole on the cuff unit side can also be set longer. Therefore, reliability of the connection of the air passages can be increased.

According to the fourth invention, the air connector is disposed on the connecting surface of the body unit, and by this air connector, the air pump, the exhaust valve, and the pressure sensor of the body unit, and the air passage on the cuff unit side are connected to each other. Therefore, for example, by constituting the air connector by a soft material (soft resin or rubber, etc.) different from a casing on the body unit side (mainly made of hard resin), the air passage on the body side and the air passage on the cuff unit side can be connected without difficulty, via the air connector. When there is no air connector provided, the air passage connection hole for directly connecting the cuff unit and the case of the body unit is formed. However, in this case, it is difficult to connect the air passage connection hole on the body unit side and the air passage connection hole on the cuff unit side, because usually the case itself is constituted of the hard resin.

According to the fifth invention, the air connector is disposed on the connecting surface of the body unit, for connecting the air passage of the air pump and the exhaust valve, and the air passage of the pressure sensor, and the connection hole is provided on the end portion of this air connector for connecting to the cuff unit side. Therefore, handling of air circuits in the body unit can be decreased, and this contributes to making the body unit compact. In addition, the first connection hole to which the air pump and the exhaust valve are connected, and the second connection hole to which the pressure sensor is connected, are provided on the outer periphery of a pipe-shaped air connector, and the air passage connection hole for connecting to the cuff unit side is provided on the end portion. Therefore, the air connector can be supported at two places of the first connection hole and the second connection hole. Therefore, even when the air connector is constituted of soft tube, the air connector is not bent in the middle and the air passage connection hole of the end portion can be connected to the air passage connection hole on the cuff unit side.

In addition, the air connector is disposed in the axial direction of the cuff unit, and therefore the length can be set longer without difficulty. Therefore, an interval between the first connection hole and the second connection hole can be freely decided, and a degree of free design of an internal layout can be increased.

According to the sixth invention, the protruding part that protrudes toward a curved inner face is provided in approximately the center position of the cuff spring. Therefore, when the cuff unit is wound around the wrist, with the cuff spring appropriately fitted with a pulsation part side of the wrist, the cuff unit can be positioned between positions corresponding to two arteries passing through the pulsation part of the wrist. Therefore, when air is supplied to a bladder of the cuff unit from the air pump or the body unit, the bladder can be appropriately swollen on both sides of the protruding part, and by this swollen part, each of the two arteries of the wrist can be effectively pressed, and a measurement accuracy of a blood pressure can be improved.

In addition, by forming this protruding part on the inner surface of the cuff spring, the air connector is accommodated in the recess portion formed on the outer surface side. Therefore, the air connector can be accommodated without taking up wasteful spaces. Namely, an accommodating space for the air connector needs not to be taken largely on the body unit side, thus making it possible to suppress increase of a volume of the body unit. This contributes to making the sphygmomanometer compact.

### DESCRIPTION OF THE PREFERRED EMBODIMENTS

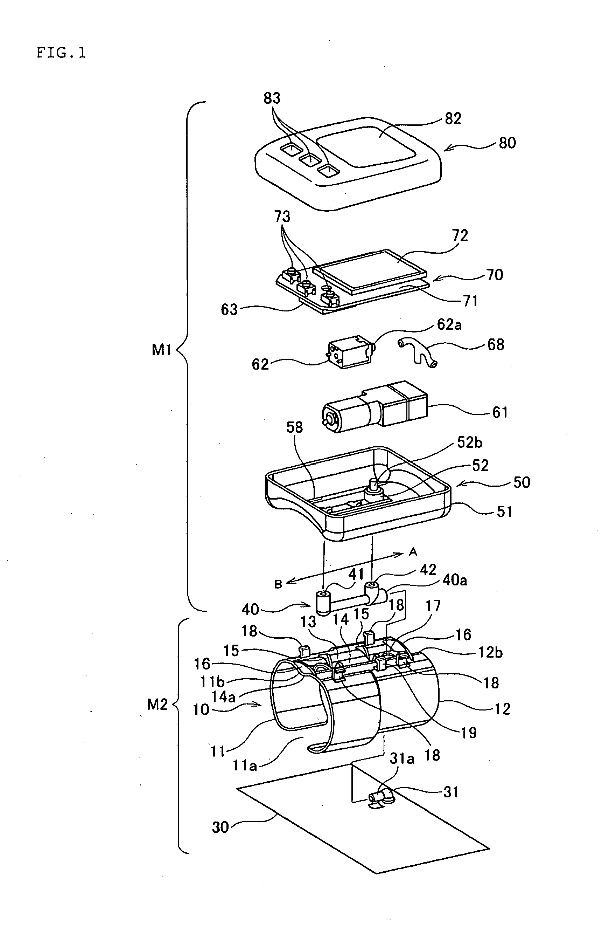

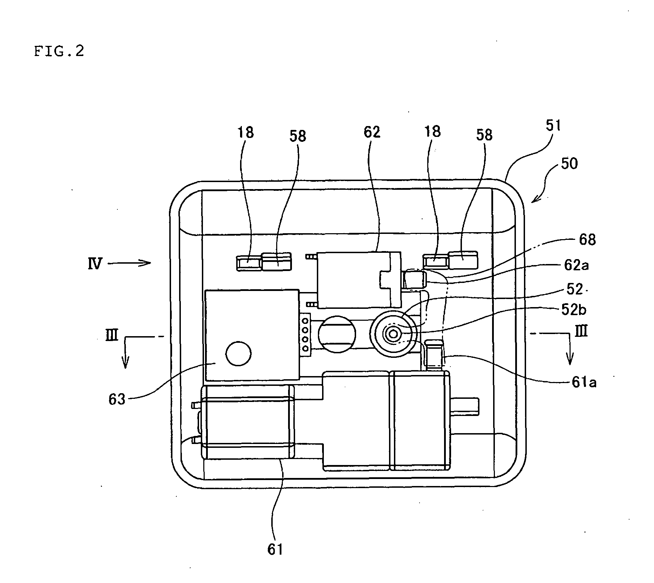

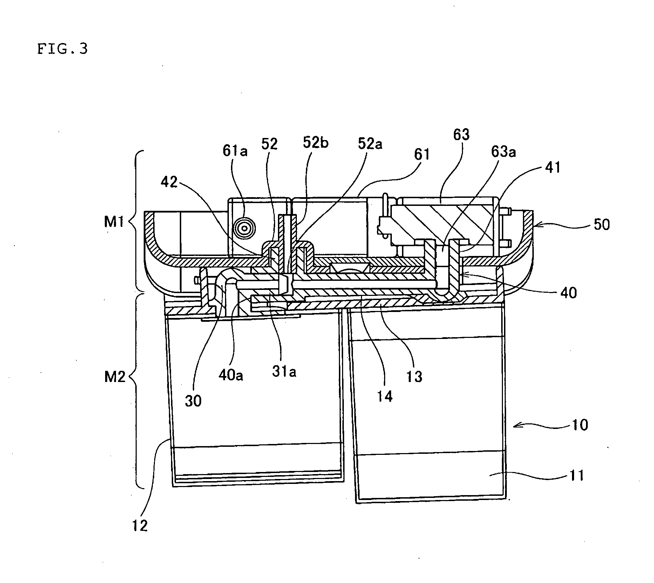

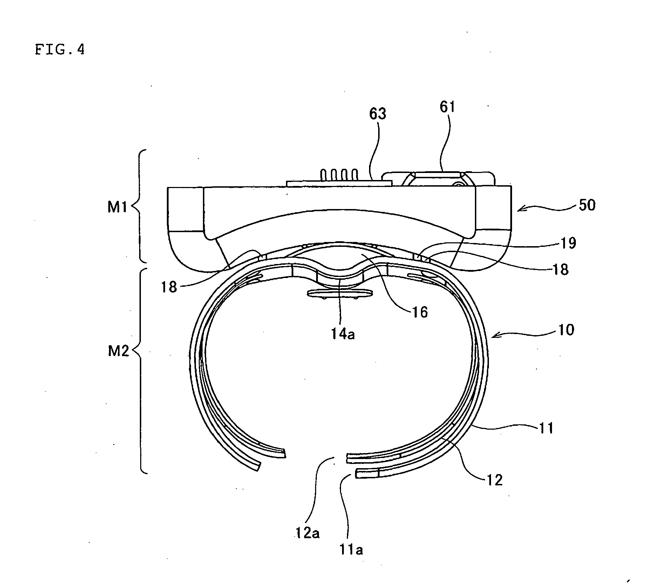

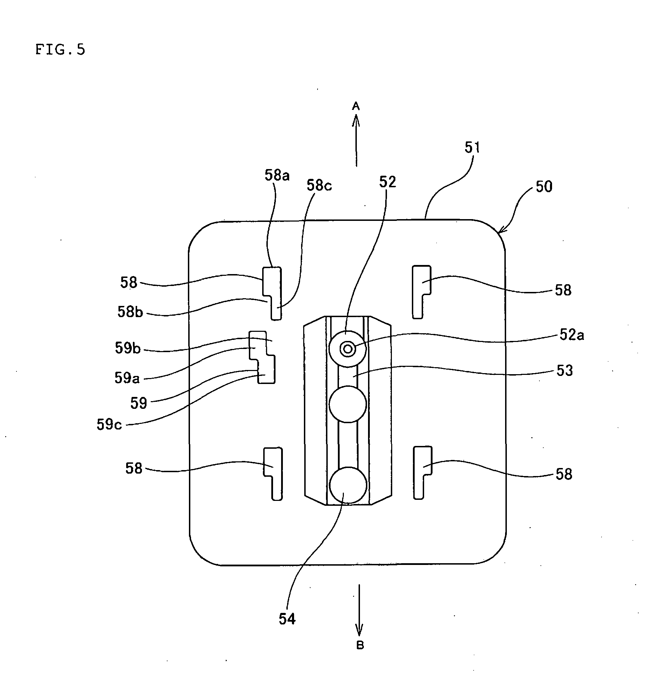

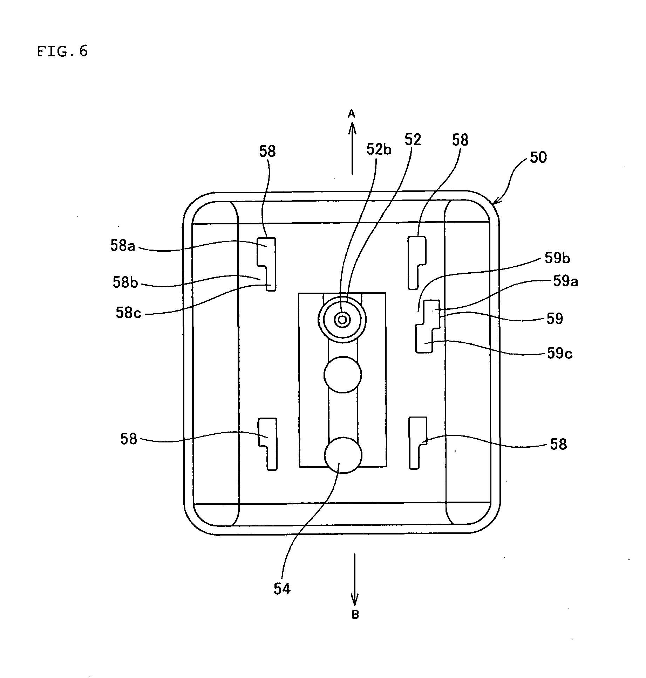

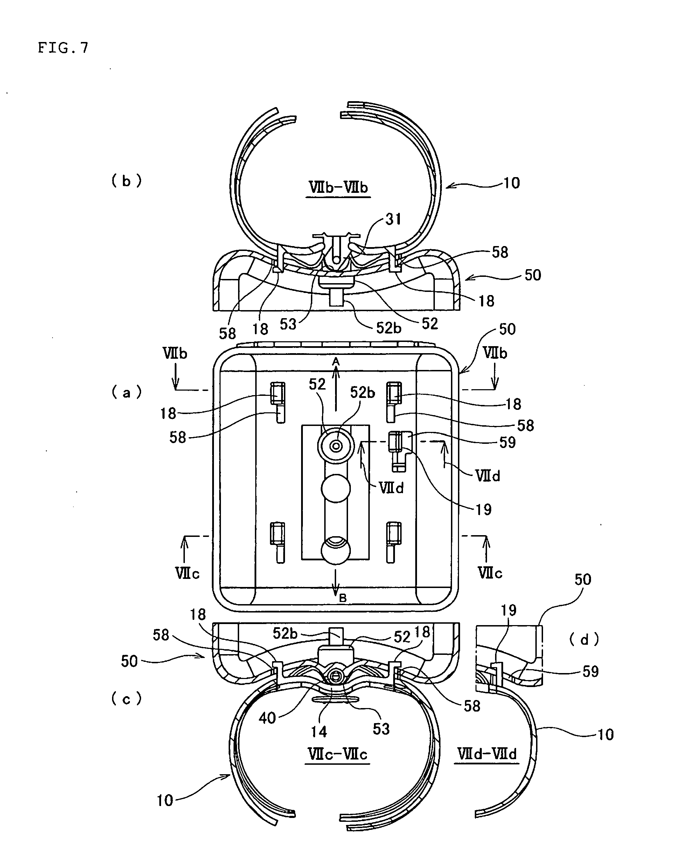

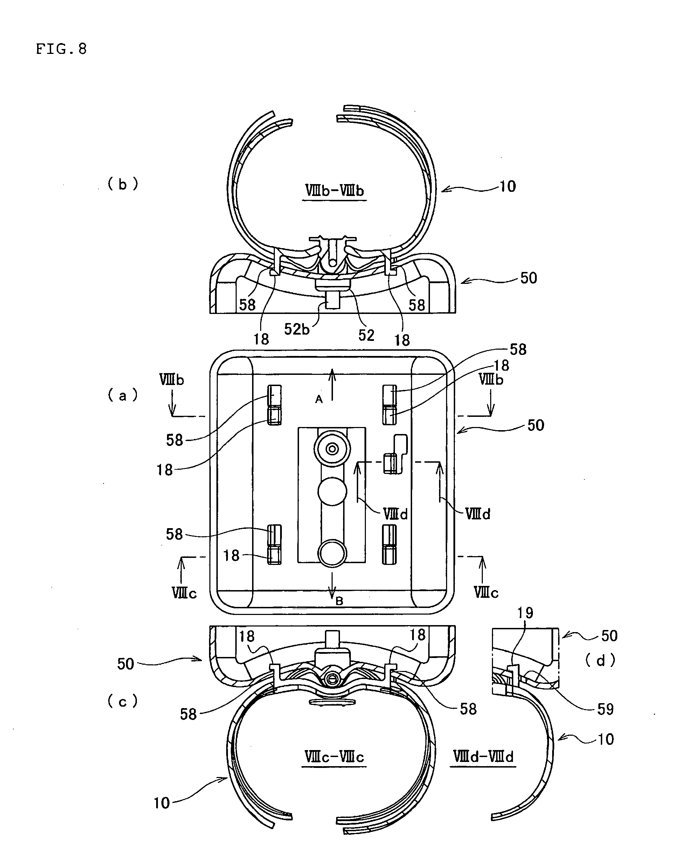

Hereinafter, embodiments of the present invention will be explained, with reference to the drawings. FIG. 1 is an exploded perspective view of a wrist sphygmomanometer according to an embodiment, FIG. 2 is a plan view illustrating a main layout of inside of a body unit of this sphygmomanometer, FIG. 3 is a sectional view of FIG. 2 taken along the line III-III shown by arrow, FIG. 4 is a view of IV in FIG. 2 shown by arrow, FIG. 5 is a lower surface view of a lower case of the body unit, FIG. 6 is an upper surface view of this lower case, and FIG. 7 is a view illustrating an engaging relation when an engagement hook on the cuff unit side is inserted into an engagement hole on the body unit side, wherein FIG. 7A is a plan view viewed from above, FIG. 7B is a sectional view of FIG. 7A taken along the line VIIb-VIIb shown by arrow, FIG. 7C is a sectional view of FIG. 7A taken along the line VIIc-VIIc shown by arrow, FIG. 7D is a sectional view of FIG. 7A taken along the line VIId-VIId, FIG. 8 is a view illustrating a relation between the engagement hook and the engagement hole when they are engaged in a coming-off prevention state, FIG. 8A is a plan view viewed from above, FIG. 8B is a sectional view of FIG. 8A taken along the line VIIIb-VIIIb shown by arrow, FIG. 8C is a sectional view of FIG. 8A taken along the line VIIIc-VIIIc shown by arrow, and FIG. 8D is a sectional view of FIG. 8A taken along the line VIIId-VIIId shown by arrow.

As shown in FIG. 1, this wrist sphygmomanometer is formed by integrally connecting a cuff unit (cuff band) M2 wound around a wrist, to a body unit (sphygmomanometer body) M1. The cuff unit M2 incorporates a bladder (air bag) 30, and by supplying air to the bladder 30 and making it swollen, an artery passing through the wrist is pressurized and a blood pressure is measured. A measurement result is displayed on a display part 82 on an upper surface of this body unit M1, and therefore a measuring person can know the measurement result by observing it by his/her own eye.

The cuff unit M2 includes a bladder (air bag) 30 disposed along a curve of a cuff spring 10, inside of a curve-shaped cuff spring 10 made of a hard material, and an entire body thereof is covered with an outer bag (not shown) made of fabric, etc. A bladder connector 31, being an exit/entry of air, is provided on one end of the bladder 30, and the bladder connector 31 is protruded to outside of the cuff spring 10 through a through hole 17 of the cuff spring 10 (see FIG. 3). An air passage connection hole 31a directed to an arrow B side in an axial direction of the cuff spring 10 is provided in the bladder connector 31.

The cuff spring 10 has two spring parts 11 and 12 of C-ring types with large diameter and small diameter having openings 11a and 12a (see FIG. 4) at a part in a peripheral direction, and opposite side parts 11b and 12b of the openings 11a and 12a are connected to each other as a mount part 13. Then, an outer surface of this mount part 13 is formed into a connecting surface to be connected to the body unit M1, and an arch-shaped protruding part 14a that protrudes toward an inner surface side of the mount part 13 is provided on an outer surface (connecting surface) of the mount part 13, to thereby form a recess portion 14 for accommodating the air connector 40 (as will be described later).

This recess portion 14 is provided along the axial direction of the cuff spring 10, and is reinforced by four ribs 16, 15, in two places of both end parts and an intermediate part in the axial direction. A circular cutout for accommodating the air connector 40 is formed in the intermediate rib 15. The through hole 17, through which the bladder connector 31 protrudes, is provided at a position closer to one end side in the axial direction.

In addition, engagement hooks 18 are protruded from the outer surface (connecting surface) of the mount part 13 of the cuff spring 10, so as to be positioned at four vertexes of a rectangle. The claws of the tip end of the engagement hooks 18 are all directed to outside, respectively. In addition, separately from these four engagement hooks 18, one lock claw 19 having the same shape as the shape of the engagement hook 18 is protruded. The claw of the tip end of this lock claw 19 is directed inward.

Note that any number of the engagement hooks 18 may be set as the number of the engagement hooks 18, provided that a plurality of engagement hooks 18 are arranged on the connecting surface in a balanced manner. Also, three engagement hooks 18 and one lock claw 19 may be arranged, so as to be positioned at four vertexes of the rectangle. Further, the direction of the claw of the tip end is not particularly limited.

Meanwhile, the body unit M1 incorporates an air pump 61, being a blood pressure measurement function component, an exhaust valve 62, a pressure sensor 63, and a substrate unit 70, etc, inside of a casing composed of a lower case 50 and an upper case 80. The pressure sensor 63 is attached to a lower surface of a circuit substrate 71 of a substrate unit 70, and a display panel 72 such as a liquid crystal and switches 73 are attached to the upper surface of the circuit board 71. Then, by combining the upper case 80 and the lower case 50 into one, the switches 73 are positioned below an operation part 83, and the display panel 72 is positioned below a display window 82.

The lower case 50 has a box-shaped case body 51, with a bottom wall curved in an arch shape corresponding to a curve of an outer surface of the cuff spring 10, and two ports for connecting the air circuits are provided on the bottom wall. One of the ports is provided as a through hole 54 through which a second connection hole 41 of the air connector 40 connected to the pressure sensor 63 is protruded. In addition, the other port is provided as an air passage connection part 52 connected to the air pump 62 and the exhaust valve 62. A plug-shaped lower side connection hole 52a connected to a first connection hole 42 of the air connector 40 is protruded from the lower side of the air passage connection part 52, and a plug-shaped upper side connection hole 52b connected to the air circuit is protruded from the upper side. This upper side connection hole 52b is connected to the air pump 61 and both air connection holes 61a and 62a of the exhaust valve 62, via a T-shaped tube 68.

In addition, the lower surface of the bottom wall of the lower case 50 is formed into the connecting surface connected with the cuff unit M2, and an air connector accommodating groove 53 for accommodating the air connector 40 is provided on the connecting surface. An upper half of the air connector 40 is engaged with this accommodating groove 53.

The air connector 40 is mounted on the lower surface (connecting surface) of the lower case 50 of the body unit M1, and is constituted as a pipe-shaped component made of soft resin, with an opening provided on longitudinal one end, being the air passage connection hole 40a on the body unit M1 side, and the other end closed, having on the outer periphery the first connection hole 42 connected to the air pump 61 and the exhaust valve 62 equipped in the body unit M1, and the second connection hole 41 connected to the pressure sensor 63 equipped in the body unit M1. This air connector 40 is disposed approximately in parallel to the axial direction of the cuff unit M2, and the air passage connection hole 40a is directed to direction A shown by arrow opposite to direction B shown by arrow.

Then, the air passage connection hole 40a on the body unit M1 side and the air passage connection hole 31a on the cuff unit M2 side are engaged with each other by making the body unit M1 slide relative to the cuff unit M2 in the direction A shown by arrow (first direction), and this engagement is released by making them slide in the direction B shown by arrow (second direction).

In addition, an engagement hole 58 and a lock hole 59 for detachably engaging with the engagement hook 18 and the lock claw 19 on the cuff unit M2 side are formed on the bottom wall of the lower case 50. The body unit M1 and the cuff unit M2 are connected to each other by making the engagement hook 18 and the engagement hole 58 engaged with each other, and making the lock claw 19 and the lock hole 59 engaged with each other.

As shown in FIG. 5 and FIG. 6, each engagement hole 58 has a through hole part 58a through which the engagement hook 18 can be extracted and inserted from/into the engagement hole 58, by relatively moving the body unit M1 and the cuff unit M2 in the direction orthogonal to the connecting surface; and a slipoff preventive hole part 58c with locking wall 58b for preventing coming-off of the engagement hook 18 by making the body unit M1 and the cuff unit M2 relatively slide in the first direction (direction A shown by arrow for the body unit M1) parallel to the connecting surface.

The lock claw 19 on the cuff unit M2 side and the lock hole 59 on the body unit M1 side constitute a lock mechanism for inhibiting the movement of the body unit M1 and the cuff unit M2 in the second direction (direction B shown by arrow for the body unit M1) opposite to the first direction, in a state of moving the engagement hook 18 to the slipoff preventive hole part 58c with locking wall 58b of the engagement hole 58.

. The lock hole 59 has an insertion hole part 59a with locking wall 59b for receiving the lock claw 19 by utilizing its bending property, when the body unit M1 and the cuff unit M2 are relatively moved in the direction orthogonal to the connecting surface; and a lock hole part 59c for inhibiting the movement of the body unit M1 and the cuff unit M2 in the second direction of the body unit M1 and the cuff unit M2 (direction B for the body unit M1) by making them abut on the locking wall 59b of the insertion hole part 59a with locking wall 59b, in such a manner that when the body unit M1 and the cuff unit M2 are relatively slid in the first direction parallel to the connecting surface (direction A shown by arrow for the body unit M1) and the engagement hook 18 is inserted into the slipoff preventive hole part 58c with locking wall 58b of the engagement hole 58, the lock claw 19 is received and in this state, the body unit M1 and the cuff unit M2 are relatively moved in the second direction (direction B shown by arrow for the body unit M1), the body unit M1 and the cuff unit M2 are abut on the locking wall 59b of the insertion hole part 59a with locking wall 59b, thereby inhibiting the movement of the body unit M1 and the cuff unit M2 in the second direction (direction B shown by arrow for the body unit M1).

Note that the first direction and the second direction are set approximately in parallel to the axial direction of the cuff unit M2, and arrow A in the figure indicates the first direction for the body unit M1, and arrow B in the figure indicates the second direction for the body unit M1, respectively.

Next, an action will be explained.
First, when the body unit M1 is assembled, the air pump 61 and the exhaust valve 62 are disposed in the loser case 50, and by the T-shaped tube 68, the upper side connection hole 52b of the air connection part 52 and both connection holes 61a and 62a of the air pump 61 and the exhaust valve 62 are connected.

Next, the substrate unit 70 with pressure sensor 63 is set thereon, and further covered with the upper case 80 thereon, thus combining the upper case 80 and the lower case 50 into one. Note that the substrate unit 70 may be attached to the lower surface of the upper case 80 in advance.

Subsequently, the air connector 40 is disposed on the lower surface of the lower case 50, and the first connection hole 42 of the air connector 40 is engaged with the lower side connection hole 52b of the air connection part 52 of the lower case 50, and the second connection hole 41 of the air connector 40 is engaged with the connection hole 63a of the pressure sensor 63 through the through hole 54 of the lower case 50. Thus, the air connector 40 is engaged with the air connector accommodating groove 53 of the lower surface of the lower case 50, and the air passage connection hole 40a of the air connector 40 is set so as to be directed in the direction A shown by arrow. Thus, the body unit M1 is assembled.

Next, a case of connecting the body unit M1 and the cuff unit M2 will be explained.
In this case, the connecting surface (lower surface of the lower case 50) of the body unit M1 is made to approach the connecting surface (upper surface of the mount part 13 of the cuff spring 10) of the cuff unit M2, while making these connecting surfaces abut on each other) (this is called a "first operation"), and as shown in Fig. 7, the engagement hook 18 is positioned to a through hole 58a (see Fig. 5 and Fig. 6) of the engagement hole 58, thereby inserting the engagement hook 18 into the engagement hole 58 through a through hole part 58a. Simultaneously, the lock claw 19 is inserted into an insertion hole part 58a with the locking wall 59b of the lock hole 59, by utilizing this bending property.

Subsequently, in this insertion state, the body unit M1 is slid in a direction A shown by arrow with respect to the cuff unit M2 (this is called a "second operation"). Then, the engagement hook 18 can be moved to the slipoff preventive hole 58c (see Fig. 5 and Fig. 6) with the locking wall 58b, thereby engaging the engagement hook 18 and the locking wall 58b, thus making it possible to prevent the coming-off of the engagement hook 18.

In addition, in this stage, the lock claw 19 is moved to the lock hole part 59c, and the lock claw 19 abuts on the locking wall 59b, thereby inhibiting return of the body unit M1 in the direction B shown by arrow. Namely, the engagement hook 18 can not return to the through hole part 58a, and a coming-off prevention state can be surely maintained.

In addition, by the operation of sliding the body unit M1 in the direction A shown by arrow, with respect to the cuff unit M2, the air passage connection hole 40a at the body unit M1 side and the air passage connection hole 31a at the cuff unit M2 side can be connected to each other, and thus, the body unit M1 and the cuff unit M2 are completely connected to each other.

In this case, by performing the second operation after performing the first operation, the prevention of the coming-off of the engagement hook 18 can be surely performed, and therefore as long as the engagement between the lock claw 19 and the lock hole 59 is not released, the connection between the body unit M1 and the cuff unit M2 is not released, even if separating them with a strong force. Namely, even if a releasing force acts between the body unit M1 and the cuff unit M2 in a direction orthogonal to the connecting surface, the body unit M1 and the cuff unit M2 are not released from each other, as long as the engagement hook 19 does not move to the through hole part 58a, because the engagement hook 18 is engaged with the slipoff preventive hole part 58c. Accordingly, in order to make the engagement hook 18 slip off from the engagement hole 58, two different directional movement (reverse operation to the second operation and reverse operation to the first operation) must be continuously caused, and when the body unit M1 and the cuff unit M2 are connected once, they are hardly released from each other.

In addition, next to the first operation of inserting the engagement hook 18 into the engagement hole 58, and the second operation of relatively sliding the body unit M1 and the cuff unit M2 in a direction parallel to the connecting surface. Then, at this time first, the air passage connection hole 40a at the body unit M1 side is connected to the air passage connection hole 31a at the cuff unit M2 side, and therefore while guiding sliding operation of the engagement hook 18, the air passage connection holes 40a, 31a can be connected to each other, thus making it easy to connect the air circuits. Namely, positioning of the body unit M1 and the cuff unit M2 must be performed only when positioning of the engagement hook 18 and the engagement hole 58 are performed, and the connection can be completed only by performing automatic guiding operation when the air passage connection holes 40a, 31a are connected to each other. Accordingly, a connection work between the body unit M1 and the cuff unit M2 can be facilitated.

In addition, when the connection between the body unit M1 and the cuff unit M2 is released, first, the engagement between the lock claw 19 and the lock hole 59 is released. As a method thereof, the cuff spring 10 with the lock claw 19 is bent inward (in a direction of smaller diameter) with a stronger force. Then, a claw at the tip end is displaced outward and is released from the locking wall 59b, because the claw at the tip end of the lock claw 19 is directed inward. Therefore, while allowing the cuff spring 10 to bend as it is, the lock claw 19 is extracted from the lock hole 59, and simultaneously, and the body unit M1 and the cuff unit M2 are slid in the second direction (direction B shown by arrow for the body unit M1).

Then, the engagement hook 18 is moved to the through hole part 58a from the slipoff preventive hole part 58c with the locking wall 58b. In addition, simultaneously, the air passage connection hole 40a at the body unit M1 side is released from the air passage connection hole 31a at the cuff unit M2 side. Accordingly, in this state, when the body unit M1 and the cuff unit M2 are relatively moved in a releasing direction orthogonal to the connecting surface, the engagement hook 18 can be completely extracted from the engagement hole 58, and the connection between the body unit M1 and the cuff unit M2 can be released. As a result, for example, even in a case of the maintenance and replacement of the cuff unit M2, by separating the cuff unit M2 from the body unit M1, the work can be simply performed.

Thus, by strongly bending the cuff spring 10 inward (by adding the operation not performed in normal use), the lock claw 19 can be released from the lock hole 59. Therefore, without detaching the upper case 80, namely, without opening the upper case 80, the body unit M1 and the cuff unit M2 can be separated from each other, and a detachment work can be facilitated.

Incidentally, when the cuff unit M2 is wound around the wrist in the normal use, an outward bending force acts in a direction of increasing the diameter of the cuff spring 10. Therefore, an inward claw of the tip end of the lock claw 19 inclines inside, and is more strongly engaged with the locking wall 59b. Accordingly, there is no possibility that the lock claw 19 is released from the lock hole 59.

In addition, according to this embodiment, only by forming a combination of the lock claw 19 and the lock hole 59 on the connecting surface of the body unit M1 and the cuff unit M2, in the same way as the combination of the engagement hook 18 and the engagement hole 58, a lock mechanism can be constituted. Therefore, design and processing can be facilitated. In addition, insertion of the lock claw 19 into the lock hole 59 can be performed simultaneously with an inserting operation of the engagement hook 18 into the engagement hole 58, and therefore there is no trouble generated, in operation. Also, by utilizing the bending property of the lock claw 19 itself, the lock claw 19 is inserted into the insertion hole part 59a with locking wall 59b of the lock hole 59. Therefore, temporary locking action between the locking wall 58b and the lock claw 19 can be expected, and transition from the aforementioned first operation to the second operation can be smoothly performed.

In addition, the direction of slide operations of the body unit M1 and the cuff unit M2 for engaging and releasing the engagement hook 18 and the engagement hole 58 is set in an axial direction of the cuff unit M2. Therefore, a strong connection structure strong in the movement in the lateral direction orthogonal to the axial direction (vertical direction) can be realized. This is because an idle dimension of the engagement hole 58 (particularly the slipoff preventive hole part 58c with locking wall 58b) can be set small as much as possible, in the lateral direction orthogonal to the direction of slide operation.

Therefore, for example, when actually using the sphygmomanometer by fitting the cuff unit M2 around the wrist, positional adjustment is performed while rotating the cuff unit M2 around the wrist in many cases. At that time, lateral force is added to the body unit M1 in many cases, and even in such a case of adjustment operation, the connection state between the body unit M1 and the cuff unit M2 can be maintained without any rattling.

In addition, when the body unit M1 and the cuff unit M2 are slid in the vertical direction (axial direction), the length of the connecting surface of the body unit M1 and the cuff unit M2 can be set, so that a vertical length is longer than a lateral length. Therefore, sliding stroke can be set longer. This is because the cuff unit M2 has a curvature in a direction (lateral direction) orthogonal to the axial direction for winding it around the wrist, and it is difficult to ensure the length of the connecting surface in the lateral direction. Accordingly, longer sliding stroke can be obtained, and therefore an engaging width (engaging margin) of the engagement hook 18 and the locking wall 58b can be taken larger, thus making it possible to significantly contribute to a strength of engagement. In addition, it is possible to take a greater engagement length of the air passage connection hole 40a at the body unit M1 side and the air passage connection hole 31a at the cuff unit M2 side, and therefore reliability of connection of the air passages can be enhanced.

In addition, according to this embodiment, the air connector 40 connecting the air passage of the air pump 61 and the exhaust valve 62, and the air passage of the pressure sensor 63, is disposed on the connecting surface of the body unit M1, and the air passage connection hole 40a is provided on the end portion of this air connector 40, so as to be connected to the cuff unit M2 side. Therefore, management of air circuits within the body unit M1 can be reduced, and this contributes to making the body unit M1 compact. In addition, there are provided the first connection hole 42 to be connected to the air pump 61 and the exhaust valve 62, and the second connection hole 41 to be connected to the pressure sensor 63, on the outer periphery of a pipe-shaped air connector 40, and the air passage connection hole 40a is provided on the end portion to be connected to the cuff unit M2 side. Therefore, the air connector 40 can be supported at two places of the first connection hole 42 and the second connection hole 41, and even when the air connector 40 is constituted of a soft tube, the air passage connection hole 40a of the end portion can be easily connected to the air passage connection hole 31a at the cuff unit M2 side, without being buckled on the way.

In addition, since the air connector 40 is disposed in the axial direction of the cuff unit M2, and therefore the length can be set longer without difficulty. Therefore, an interval between the first connection hole 42 and the second connection hole 41 can be freely determined, and a free degree in a design of an internal layout of the body unit M1 can be enhanced.

Also, according to this embodiment, the protruding part 14a protruding to the curved inner surface side is provided, in approximately peripheral center position of the cuff spring 10. Therefore, when the cuff unit M2 is wound around the wrist, with the cuff spring 10 properly matched with the pulsation part side of the wrist, the cuff unit M2 can be positioned between positions corresponding to two arteries passing through the inside of the pulsation part of the wrist. Therefore, when air is supplied to the bladder 30 of the cuff unit M2 from the air pump 61 of the body unit M1, the bladder 30 can be suitably swollen at both sides of the protruding part 14a, and each of the two arteries of the wrist can be effectively pressurized by this swollen part, and a measurement accuracy of the blood pressure can be improved.

In addition, by forming this protruding part 14a on the side of the inner surface of the cuff spring 10, the air connector 40 is accommodated in the recess portion 14 formed on the outer surface side, and therefore the air connector 40 can be accommodated without generating a useless space. Namely, a large accommodation space (air connector accommodation groove 53) for the air connector 40 at the body unit M1 side is not required at the body unit M1 side, therefore increase of a volume of the body unit M1 can be suppressed, and this contributes to making the sphygmomanometer compact.

Note that in the aforementioned embodiment, the lock mechanism is constituted of the lock claw 19 and the lock hole 59. However, any lock mechanism can be adopted, if the body unit M1 is locked so as not to return in the direction B shown by arrow, with the body unit M1 moved in the direction A shown by arrow.

In addition, the aforementioned embodiment shows a case in which there are provided two connection holes such as the first connection hole 42 connected to the air pump 61 and the exhaust valve 62, and the second connection hole 41 connected to the pressure sensor 63. However, it may be preferable that these two connection holes are put together to form the air connector 40 in an L-shaped coupling component. However, in this case, the air passage must be branch-connected to the air pump 61, the exhaust valve 62, and the pressure sensor 63, inside of the body unit M1.

### Brief Description of the Drawings

Fig. 1 is an exploded perspective view of a wrist sphygmomanometer according to an embodiment of the present invention;
Fig. 2 is a plan view illustrating a principal layout of inside of the body unit of this sphygmomanometer;
Fig. 3 is a sectional view taken along the line III-III of Fig. 2;
Fig. 4 is a view shown by arrow IV of Fig. 2;
Fig. 5 is a lower surface view of a lower case of this unit;
Fig. 6 is an upper surface view of this lower case;
Fig. 7 is a view illustrating an engagement relation at the time of inserting an engagement hook on the cuff unit side into an engagement hole on the body unit side, (a) is a plan view viewed from above, (b) is a sectional view shown by arrow taken along the line VIIb-VIIb, (c) is a sectional view shown by arrow taken along the line VIIc-VIIc, and (d) is a sectional view shown by arrow taken along the line VIId-VIId.
Fig. 8 is a view illustrating the engagement relation when the engagement hook and the engagement hole are engaged with each other in a slipoff preventive state, by relatively sliding the body unit and the cuff unit, and (a) is a plan view viewed from above, (b) is a sectional view shown by arrow taken along the line VIIIb-VIIIb, (c) is a sectional view shown by arrow taken along the line VIIIc-VIIIc, and (d) is a sectional view shown by arrow taken along the line VIIId-VIIId.

### (Description of Signs and Numerals)

- M1: Body unit
- M2: Cuff unit
- A: First direction
- B: Second direction
- 10: Cuff spring
- 14: Recess portion
- 14a: Protruding part
- 18: Engagement hook
- 19: Lock claw (lock mechanism)
- 31a: Air passage connection hole on the cuff unit side
- 40: Air connector
- 40a: Air passage connection hole on the body unit side
- 41: Second connection hole
- 42: First connection hole
- 58: Engagement hole
- 58a: Through hole part
- 58b: Locking wall
- 58c: Slipoff preventive part
- 59: Lock hole (lock mechanism)
- 59a: Insertion hole part
- 59b: Locking wall
- 59c: Locking hole part
- 61: Air pump
- 62: Exhaust valve
- 63: Pressure sensor

## Claims

1. A sphygmomanometer composed of a body unit (M1) and a cuff unit (M2) connected to a bottom surface of this body unit, with plural sets of engagement hooks (18) and engagement holes (58) formed on one and the other side of a connecting surface of the body unit and the cuff unit respectively to be freely engaged with one another, so that by engaging the engagement hooks and the engagement holes with one another, the body unit and the cuff unit are connected to each other, and also an air passage connection hole (40a) on the body unit side and an air passage connection hole (31a) on the cuff unit side are connected to each other, wherein
the engagement holes comprise passage holes (58a) through which the engagement hooks can be inserted and extracted into/from the engagement holes, by relatively moving the body unit and the cuff unit in a direction orthogonal to the connecting surface; and slip-off prevention holes (58c) having engagement walls for preventing slip-off of the engagement hooks, by relatively sliding the body unit and the cuff unit in a first direction parallel to the connecting surface, with the engagement hooks inserted into the passage holes,
a lock mechanism (19, 59) is provided in the body unit and the cuff unit for inhibiting a movement of the body unit and the cuff unit in a second direction opposite to the first direction, with the engagement hooks moved to the slip-off prevention holes having the engagement walls of the engagement holes, the air passage connection holes on the body unit side and the air passage connection holes on the cuff unit side are engaged with one another by relatively sliding the body unit and the cuff unit in the first direction, with the engagement hooks inserted into the engagement holes, wherein the engagement can be released by relatively sliding the body unit and the cuff unit in the second direction,
the lock mechanism is composed of a set of a lock claw (19) and a lock hole (59) provided at one side and the other side of the connecting surface of the body unit and the cuff unit respectively,
the lock hole comprises an insertion hole part (59a) with locking wall for receiving the lock claw by utilizing a bending property of the lock claw, when the body unit and the cuff unit are relatively moved in the direction orthogonal to the connecting surface; and a locking hole part (59c) for inhibiting the movement of the body unit and the cuff unit in a second direction in a state in which the lock claw is received after the body unit and the cuff unit are relatively slid in the first direction parallel to the connecting surface and the engagement hooks are inserted into slip-off prevention holes having engagement wall of the engagement holes, and further in this state the lock claw abuts against locking wall (59b) of the insertion hole part with locking wall when the body unit and the cuff unit are attempted to be relatively moved in the second direction.

2. The sphygmomanometer according to claim 1, wherein the first direction and the second direction are set in an axial direction of the cuff unit.

3. The sphygmomanometer according to claim 1 or 2, wherein there is provided an air connector (40) on the connecting surface of the body unit, the air connector having a connection hole at one end side as an air passage connection hole on the body unit side, and at the other end side a connection hole connected to an air pump (61), an exhaust valve (62), and a pressure sensor (63) equipped in the body unit.

4. The sphygmomanometer according to claim 3, wherein the air connector is constituted of a tubular component having a first connection hole (42) connected to the air pump and the exhaust valve equipped in the body unit and a second connection hole (41) connected to the pressure sensor equipped in the body unit in an outer periphery of the tubular component, with an opening provided on one longitudinal end of the tubular component, being the air passage connection hole (40a) on the body unit side, and the other end closed, and the air connector with this structure is disposed parallel to an axial direction of the cuff unit on the connecting surface of the body unit.

5. The sphygmomanometer according to claim 4, wherein a protruding part (14a) that protrudes toward a curved inner surface side of a curve-shaped cuff spring (10) is provided in a center position in the peripheral direction of said curve-shaped cuff spring (10) constituting the cuff unit, and the air connector is accommodated in a recess portion formed on an outer surface side of the cuff spring by providing the protruding part.

## Patentansprüche

1. Blutdruckmesser, zusammengesetzt aus einer Körpereinheit (M1) und einer Manschetteneinheit (M2), die mit einer unteren Oberfläche dieser Körpereinheit verbunden ist, mit mehreren Sätzen von Eingriffshaken (18) und Eingriffslöchern (58), die an einer und der anderen Seite einer Verbindungsoberfläche der Körpereinheit bzw. der Manschetteneinheit derart gebildet sind, dass sie frei miteinander eingreifen, so dass durch miteinander Eingreifen der Eingriffshaken und der Eingriffslöcher die Körpereinheit und die Manschetteneinheit gegenseitig verbunden sind und ebenso ein Luftdurchgangsverbindungsloch (40a) an der Seite der Körpereinheit und ein Luftdurchgangsverbindungsloch (31a) an der Seite der Manschetteneinheit gegenseitig verbunden sind, wobei
die Eingriffslöcher Durchgangslöcher (58a) umfassen, durch welche die Eingriffshaken in/aus die/den Eingriffslöchern eingeführt und herausgezogen werden können, und zwar durch relatives Bewegen der Körpereinheit und der Manschetteneinheit in eine Richtung, die orthogonal zu der Verbindungsoberfläche ist; und Abgleitverhinderungslöcher (58c), die Eingriffswände haben, um ein Abgleiten der Eingriffshaken durch relatives Schieben der Körpereinheit und der Manschetteneinheit in eine erste Richtung parallel zu der Verbindungsoberfläche zu verhindern, wobei die Eingriffshaken in die Durchgangslöcher eingeführt sind,
ein Schließmechanismus (19, 59) in der Körpereinheit und der Manschetteneinheit bereitgestellt ist, um eine Bewegung der Körpereinheit und der Manschetteneinheit in einer zweiten Richtung entgegengesetzt zu der ersten Richtung zu verhindern, wobei die Eingriffshaken zu den Abgleitverhinderungslöchern, die die Eingriffswände der Eingriffslöcher haben, bewegt werden, die Luftdurchgangsverbindungslöcher an der Seite der Körpereinheit und die Luftdurchgangsverbindungslöcher an der Seite der Manschetteneinheit miteinander eingreifen durch relatives Schieben der Körpereinheit und der Manschetteneinheit in die erste Richtung, wobei die Eingriffshaken in die Eingriffslöcher eingeführt sind, wobei das Eingreifen durch relatives Schieben der Körpereinheit und der Manschetteneinheit in die zweite Richtung gelöst werden kann,
der Schließmechanismus aus einem Satz von einem Schließgreifer (19) und einem Schließloch (59) zusammengesetzt ist, die an einer Seite und der anderen Seite der Verbindungsoberfläche der Körpereinheit bzw. der Manschetteneinheit bereitgestellt sind,
das Schließloch einen Einfügungslochteil (59a) mit Schließwand zum Empfangen des Schließgreifers unter Nutzung einer Biegeeigenschaft des Schließgreifers, wenn die Körpereinheit und die Manschetteneinheit relativ in der Richtung, die orthogonal zu der Verbindungsoberfläche ist, bewegt werden, umfasst; und einen Schließlochteil (59c) zum Verhindern der Bewegung der Körpereinheit und der Manschetteneinheit in einer zweiten Richtung in einem Zustand, in welchem der Schließgreifer empfangen ist, nachdem die Körpereinheit und die Manschetteneinheit relativ in die erste Richtung parallel zu der Verbindungsoberfläche geschoben sind und die Eingriffshaken in Abgleitverhinderungslöcher, die eine Eingriffswand der Eingriffslöcher haben, eingeführt sind, und ferner in diesem Zustand der Schließgreifer an der Schließwand (59b) des Einfügelochteils mit der Schließwand angrenzt, wenn versucht wird, die Körpereinheit und die Manschetteneinheit relativ in die zweite Richtung zu bewegen.

2. Blutdruckmesser nach Anspruch 1, wobei die erste Richtung und die zweite Richtung in einer axialen Richtung der Manschetteneinheit gesetzt sind.

3. Blutdruckmesser nach Anspruch 1 oder 2, wobei ein Luftverbinder (40) an der Verbindungsoberfläche der Körpereinheit bereitgestellt ist, wobei der Luftverbinder ein Verbindungsloch an einer Endseite als ein Luftdurchgangsverbindungsloch auf der Seite der Körpereinheit, und an der anderen Endseite ein Verbindungsloch hat, das mit einer Luftpumpe (61), einem Auslassventil (62) und einem Drucksensor (63), die in der Körpereinheit eingerichtet sind, verbunden ist.

4. Blutdruckmesser nach Anspruch 3, wobei der Luftverbinder aus einer Röhrenkomponente ausgemacht ist, die ein erstes Verbindungsloch (42), das mit der Luftpumpe und dem Abluftventil, die in der Körpereinheit vorgesehen sind, verbunden ist, und ein zweites Verbindungsloch (41) hat, das mit einem Drucksensor verbunden ist, der in der Körpereinheit in einem äußeren Umfang der Röhrenkomponente vorgesehen ist, wobei eine Öffnung an einem Längsende der Röhrenkomponente, die das Luftdurchgangsverbindungsloch (40a) auf der Seite der Körpereinheit ist, bereitgestellt ist und das andere Ende geschlossen ist, und der Luftverbinder mit dieser Struktur parallel zu einer axialen Richtung der Manschetteneinheit auf der Verbindungsoberfläche der Körpereinheit angeordnet ist.

5. Blutdruckmesser nach Anspruch 4, wobei ein vorspringender Teil (14a), der in Richtung einer gebogenen inneren Oberflächenseite einer bogenförmigen Manschettenfeder (10) in einer Mittenposition in der Umfangsrichtung der bogenförmigen Manschettenfeder (10), die die Manschetteneinheit ausmacht, bereitgestellt ist und der Luftverbinder in einem Nischenbereich, der an einer äußeren Oberflächenseite der Manschettenfeder gebildet ist, durch Bereitstellen des vorspringenden Teils aufgenommen ist.

## Revendications

1. Sphygmomanomètre composé d'une unité de corps (M1) et d'une unité de brassard (M2) raccordées à une surface de fond de cette unité de corps, avec plusieurs ensembles de crochets d'enclenchement (18) et de trous d'enclenchement (58) formés sur l'un et l'autre côtés d'une surface de raccordement de l'unité de corps et de l'unité de brassard respectivement pour être enclenchés librement l'un avec l'autre, de sorte qu'en enclenchant les crochets d'enclenchement et les trous d'enclenchement l'un avec l'autre, l'unité de corps et l'unité de brassard soient raccordées l'une à l'autre, et également un trou de raccordement de passage d'air (40a) sur le côté unité de corps et un trou de raccordement de passage d'air (31a) sur le côté unité de brassard soient raccordés l'un à l'autre, dans lequel
les trous d'enclenchement comprennent des trous de passage (58a) à travers lesquels les crochets d'enclenchement peuvent être insérés dans les trous d'enclenchement et extraits de ceux-ci, en déplaçant relativement l'unité de corps et l'unité de brassard dans une direction orthogonale à la surface de raccordement ; et des trous anti-retrait (58c) ayant des parois d'enclenchement pour empêcher un retrait des crochets d'enclenchement, en faisant coulisser relativement l'unité de corps et l'unité de brassard dans une première direction parallèle à la surface de raccordement, les crochets d'enclenchement étant insérés dans les trous de passage,
un mécanisme de verrou (19, 59) est fourni dans l'unité de corps et l'unité de brassard pour inhiber un mouvement de l'unité de corps et de l'unité de brassard dans une seconde direction opposée à la première direction, les crochets d'enclenchement étant déplacés vers les trous anti-retrait ayant les parois d'enclenchement des trous d'enclenchement, les trous de raccordement de passage d'air sur le côté unité de corps et des trous de raccordement de passage d'air sur le côté unité de brassard sont enclenchés l'un avec l'autre en faisant coulisser relativement l'unité de corps et l'unité de brassard dans la première direction, les crochets d'enclenchement étant insérés dans les trous d'enclenchement, dans lequel l'enclenchement peut être relâché en faisant coulisser relativement l'unité de corps et l'unité de brassard dans la seconde direction,
le mécanisme de verrou est composé d'un ensemble d'une mâchoire de verrou (19) et d'un trou de verrou (59) fournis d'un côté et de l'autre de la surface de raccordement de l'unité de corps et de l'unité de brassard respectivement,
le trou de verrou comprend une partie de trou d'insertion (59a) avec une paroi de verrouillage pour recevoir la mâchoire de verrou en utilisant une propriété de flexion de la mâchoire de verrou, lorsque l'unité de corps et l'unité de brassard sont déplacées relativement dans la direction orthogonale à la surface de raccordement ; et une partie de trou de verrouillage (59c) pour inhiber le mouvement de l'unité de corps et de l'unité de brassard dans une seconde direction dans un état dans lequel la mâchoire de verrou est reçue après que l'unité de corps et l'unité de brassard sont coulissées relativement dans la première direction parallèle à la surface de raccordement et que les crochets d'enclenchement sont insérés dans des trous anti-retrait ayant une paroi d'enclenchement des trous d'enclenchement, et en outre dans cet état la mâchoire de verrou vient en butée contre la paroi de verrouillage (59b) de la partie de trou d'insertion avec une paroi de verrouillage lorsque l'on tente de déplacer relativement l'unité de corps et l'unité de brassard dans la seconde direction.

2. Sphygmomanomètre selon la revendication 1, dans lequel la première direction et la seconde direction sont établies dans une direction axiale de l'unité de brassard.

3. Sphygmomanomètre selon la revendication 1 ou 2, dans lequel est fourni un raccord d'air (40) sur la surface de raccordement de l'unité de corps, le raccord d'air ayant un trou de raccordement d'un côté d'extrémité en tant que trou de raccordement de passage d'air sur le côté unité de corps, et, de l'autre côté d'extrémité, un trou de raccordement raccordé à une pompe à air (61), une vanne d'échappement (62), et un capteur de pression (63) installés dans l'unité de corps.

4. Sphygmomanomètre selon la revendication 3, dans lequel le raccord d'air est constitué d'un composant tubulaire ayant un premier trou de raccordement (42) raccordé à la pompe à air et la vanne d'échappement installés dans l'unité de corps et un second trou de raccordement (41) raccordé au capteur de pression installé dans l'unité de corps dans une périphérie externe du composant tubulaire, avec une ouverture fournie sur une extrémité longitudinale du composant tubulaire, qui est le trou de raccordement de passage d'air (40a) sur le côté unité de corps, et l'autre extrémité fermée, et le raccord d'air avec cette structure est disposé parallèlement à une direction axiale de l'unité de brassard sur la surface de raccordement de l'unité de corps.

5. Sphygmomanomètre selon la revendication 4, dans lequel une partie saillante (14a) qui fait saillie vers un côté de surface interne incurvée d'un ressort de brassard de forme incurvée (10) est fournie dans une position centrale dans la direction périphérique dudit ressort de brassard de forme incurvée (10) constituant l'unité de brassard, et le raccord d'air est logé dans une portion d'évidement formée sur un côté de surface externe du ressort de brassard en fournissant la partie saillante.
